# EUROPEAN PATENT APPLICATION

(11) **EP 2 469 272 A1**
(43) Date of publication of application: **27.06.2012**
(21) Application number: 10196504.4
(22) Date of filing: 22.12.2010
(51) Int. Cl.: G01N 27/12, G01N 33/00, G01N 33/12

(54) **Acid/Base Sensor**

(71) Applicant: Nederlandse Organisatie voor Toegepast -Natuurwetenschappelijk Onderzoek TNO, 2628 VK Delft (NL)
(72) Inventor: Smits, Edsger, 2628 VK Delft (NL); Schoo, Harmannus Franciscus Maria, 2628 VK Delft (NL)
(74) Representative: Jansen, Cornelis Marinus

(57) **Abstract**

Object of the invention is to provide a bilayer that is sensitive towards acids and bases, in particular to ammonia and/or amines, under different conditions, for example under varying temperature and/or varying humidity. This object was met by providing a bilayer sensor comprising a substrate carrying a bilayer, which bilayer comprises a first layer comprising an organic semiconductor and a second layer comprising a compound selected from the group consisting of acidic compounds and basic compounds.

## Description

The invention is directed to a sensor, a food packaging comprising a sensor, a method for preparing a sensor and the use of a sensor.

Radio-frequency identification (RFID) is the method for identifying and monitoring products (but also animals or persons) using radio waves by making use of an object, typically referred to as an RFID tag, which object is applied or incorporated into the goods.

The introduction of RFID tags has had a profound effect on the way many industries operate. Especially the enhanced traceability has proven to be an essential link to guarantee the quality and origin of goods.

Passive tags are tags which have no battery and require an external source for transmitting the signal. Current passive RFID tags are based on chips containing a memory that can be wirelessly read out to solely identify products.

An emerging application in the field of RFID tagging is the inclusion of sensors (e.g. tilt, pressure, temperature, time, e.g.) to not only confirm the goods identity but also factors possibly indicating damage during transport or storage. Such monitoring of goods by RFID is especially relevant for the transport of perishable goods, such as fruit, vegetables, meats and fish. These products need to be kept under very strict conditions (e.g. temperature, humidity) to arrive fresh at their destination. Faulty handling can result in unsellable products and also in potential health risks.

Meats and fish are the most susceptible food products due to the rapid growth of bacteria in those products. Bacterial growth on meats and fish typically causes the excretion of nitrous and sulfurous compounds, which is accompanied by strong and foul smell. For example, for fish the predominant marker indicative for bacterial growth is trimethylamine (TMA). For practical applications, low concentrations of such nitrous and sulfurous compounds, for example in the order of parts per million (ppm) need to be accurately and reliably measured.

Various ammonia and amine sensors are known in the art, but these are generally not suitable for the above-mentioned applications.

Metal oxide sensors able to detect ammonia an amine are commercially available. However, these sensors require high temperatures thus making them unsuited for passive RFID sensor tags. Furthermore, metal oxide sensors only have a limited selectivity.

Electrochemical sensors can also be used to detect ammonia and amine. However, electrochemical ammonia sensors are relatively expensive which makes them unsuitable to be used as disposables. In addition, electrochemical sensors are not suitable for the long exposure times under high humidity conditions generally encountered in food package applications. Furthermore, another disadvantage of electrochemical sensors is that their read-out may be relatively complex.

Doped polyaniline and poly(3,4-ethylene- dioxythiophene) (PEDOT) films and other organic conductors are known to have been used as ammonia sensors. A disadvantage of doped polyaniline and PEDOT sensors is their relatively small change in resistivity upon exposure to amines. Due to the small changes in resistivity, the sensor is not reliable with respect to drift and cross selectivity, especially towards water and other gases that may cause swelling.

Furthermore, polyaniline is prepared by polymerizing aniline, which is a toxic compound by inhalation and a suspected carcinogen. Due to possible health risks, a sensor comprising polyaniline is unsuitable for use in food products packaging.

An ammonia sensor comprising a polyaniline and nafion bilayer has been described by Jakubik ("Interactions of the Polyaniline and Nafion bilayer sensor structure with ammonia in a dry and wet air atmosphere", The European Physical Journal, special topics 154, 93-96 (2008)). In the bilayer described by Jakubik, polyaniline is a conducting polymer and Nafion is a super-acid catalyst, which is ion-conductive and functions as a cation exchange polymer. Jakubik shows that the resistivity of the bilayer decreases when brought into contact with ammonia. Jakubik does not describe a bilayer comprising a semiconductor.

A disadvantage of the bilayer described by Jakubik is that only small changes in resistivity were observed upon exposure to high concentrations of ammonia, *viz*. changes in resistivity smaller than 50% of the initial value. Furthermore, the changes in resistivity for various concentrations did not vary noticeably.

A further disadvantage of the bilayer described by Jakubik is that it shows a large variation in resistance of the bilayer when varying the humidity level of the atmosphere, *viz*. the resistance differs by more than a factor ten when comparing a relative humidity (RH) of about 5% and about 50%. These relatively large changes in resistance compared to the changes induced by the ammonia render the sensor highly unreliable under varying humidity.

A further disadvantage of the bilayer described by Jakubik is that one of its layers is made of polyaniline, which is, as described above, not suitable in food packaging products due to health risks.

Object of the invention is to provide a bilayer that is sensitive towards either a base, in particular ammonia or amines or towards an acid under different conditions, for example under varying temperature and/or varying humidity.

A further object of the invention is to provide a bilayer sensor wherein the bilayer shows a relatively large change in resistivity when exposed to either a base (such as ammonia and/or amine) or an acid, such as a change in resistivity by a factor 10 or more, in particular at low concentrations.

A further object of the invention is to provide a bilayer sensor that does not show large variations in selectivity and/or resistivity under varying humidity levels of the atmosphere, even under extreme humidity conditions of up to 90% humidity.

A further object of the invention is to provide a bilayer sensor which can be safely used in food applications.

At least one of the above objects has been met by providing a bilayer sensor comprising a substrate carrying a bilayer, which bilayer comprises a first layer comprising an organic semiconductor and a second layer comprising a compound selected from the group consisting of acidic compounds and basic compounds.

The compound that the sensor of the invention intends to detect may be referred to as the analyte. In case the second layer comprises an acidic compound, the analyte is a base (basic analyte), such as amine or ammonia. In this case, the sensor may be referred to as a base sensor or amine sensor. In case the second layer comprises a basic compound, the analyte is an acid (acidic analyte). In this case, the sensor may be referred to as an acid sensor.

It was found that the resistivity of the bilayer in the base sensor according to the invention increases when exposed to ammonia or amine. This increase was observed to be sufficiently high to provide the sensor with a high sensitivity towards a base such as amine and/or ammonia.

Furthermore, it was found that the bilayer may be suitably used under wide variations in temperature and/or humidity. Variations in such conditions only led to small variations in resistivity of the bilayer compared to the differences in resistivity caused by contacting the bilayer with the analyte.

Furthermore, it was found that the base sensor of the invention had a low cross sensitivity, *i.e.* a low sensitivity towards other compounds than amine and/or ammonia. The bilayer sensor showed no large differences in resistivity for acidic acid, methanol, isopropanol, octanol, butanol, acetone, heptane and toluene.

These findings are in contrast with the publication by Jakubik described above. The bilayer described therein is significantly less sensitive to amine and ammonia and is further sensitive to variations in humidity conditions. Difference with the present invention and Jakubik is that an organic semiconductor is used instead of an organic conductor.

Without wishing to be bound by any theory, it is believed that the increase in sensitivity of the base sensor of the invention, upon contacting the bilayer with a basic analyte such as ammonia or amine, is caused by dedoping of the interface of the bilayer. In the absence of a base, the acidic compound will provide the organic semiconductor with protons at the interface of the first and second layer. Thus, the semiconductor is doped with protons at its interface with the acid layer. As a result, the conductivity of the bilayer is increased at the interface of the first and second layer. However, when brought into contact with a base, the acidic compound will react in an acid-base reaction and loose a proton to the base, thereby dedoping the interface. Consequently, the conductivity of the bilayer will decrease and the resistivity of the bilayer will increase. This change in conductivity can be registered such that the sensor can indicate the presence of the basic analyte.

It is further believed that by using an organic conductor, as was done by Jakubik, the base conductivity of the bilayer is too high for the sensor to have a suitable sensitivity. Thus, the relatively small differences in conductivity of the bilayer caused by the doping and dedoping at the interface cannot be accurately measured when using an organic conductor. By using an organic semiconductor, which acts as an insulator in neutral state (*i.e.* when not doped and/or not exposed to an electric field), the bilayer can be provided with increased sensitivity.

The acid sensor is believed to work via the same principle as the base sensor. The difference is that the second layer of the sensor comprises a basic compound instead of an acidic compound. In the absence of an acid, the organic semiconductor will be doped. When brought into contact with an acid, the basic compound will react and take up a proton from the acid, thereby withdrawing electrons from the interface of the bilayer and thus dedoping the interface of the bilayer. Consequently, the conductivity of the bilayer will decrease and the resistivity of the bilayer will increase. This change in conductivity can be registered such that the sensor can indicate the presence of the acidic analyte.

The first layer of the sensor of the invention comprises a semiconductor. The term "semiconductor" as used herein refers to a material that obeys a space-charge-limited conduction model (as for example proposed by Mott) in undoped form and behaves as a conductor in doped form. Typically, the conductivity of the doped semiconductor is in the range of 10² - 10⁻⁹ siemens per centimeter (Ω⁻¹cm⁻¹). The conductivity of the undoped semiconductor is typically smaller than 10⁻¹⁰ siemens per centimeter (Ω⁻¹cm⁻¹). The semiconductor in undoped form may also refer to a semiconductor that is not fully pure, but have some unintentional doping. Such unintentional doping density may be below 10¹⁷ impurities per m³, preferably below 10¹⁶ impurities per m³_{.}

The semiconductor is preferably a p-type semiconductor. A p-type semiconductor is a semiconductor capable of sustaining and transporting positive free charge carriers (so-called "holes"), in particular protons. Thus, doping can be sustained using cationic doping agents such as protons. In such a case, the sensor is a base sensor.

The semiconductor may also be an n-type semiconductor. An n-type semiconductor is a semiconductor capable of sustaining and transporting negative free charge carriers (electrons). Thus, doping can be sustained using anionic doping agents such as electrons. In such a case, the sensor is an acid sensor.

The organic semiconductor is preferably a polymeric organic semiconductor. The skilled person will know which organic polymers are semiconductors and which are not. Preferably, the polymeric organic semiconductor is selected from the group consisting of polythiophenes, polyacenes, polyacrylamides, poly(triarylamine), polyfluorenes, and copolymers thereof. An advantage of a polymeric semiconductor is that the first layer can in such a case mainly consist of the semiconductor, without the need of a carrier material.

The semiconductor can also be a small organic molecule, in particular a compound selected from the group consisting of thiophenes, arylamines, acenes and pyrols. Such small organic molecules may be provided in the layer in a polymeric matrix.

In case the sensor is a base sensor, the second layer of the bilayer sensor comprises an acidic compound. An acidic compound is a compound that is capable of reacting in an acid base reaction with the intended basic analyte of the sensor, in particular with an amine and/or ammonia.

The acidic compound comprises one or more acidic groups. Preferably, such an acidic group is selected from the group consisting of sulphonic acids, carboxylic acids, acrylic acids and phosphonic acids.

The acidic compound can be an acidic polymer. An advantage of an acidic polymer is that the second layer can in such a case mainly consist of the acidic compound, without the need of a carrier material. Preferred examples of the acidic polymers are acidic fluoropolymer-copolymer, polystyrenesulfonate, sulfonated tetrafluorethylene polymers and fluorinated polystyrenesulfonate. An example of a suitable copolymer is a sulfonated tetrafluoroethylene based fluoropolymer-copolymer. Such a copolymer is known under the name Nafion.

The acidic compound can also be a non-polymeric organic acid. Such an organic acid may be dispersed in a polymeric matrix.

Preferably, the second layer is a polymer layer. However, in theory, the layer may also be a crystalline layer comprising crystalline acidic compounds. Such compounds may be applied to the first layer in dissolved form and subsequently crystallized

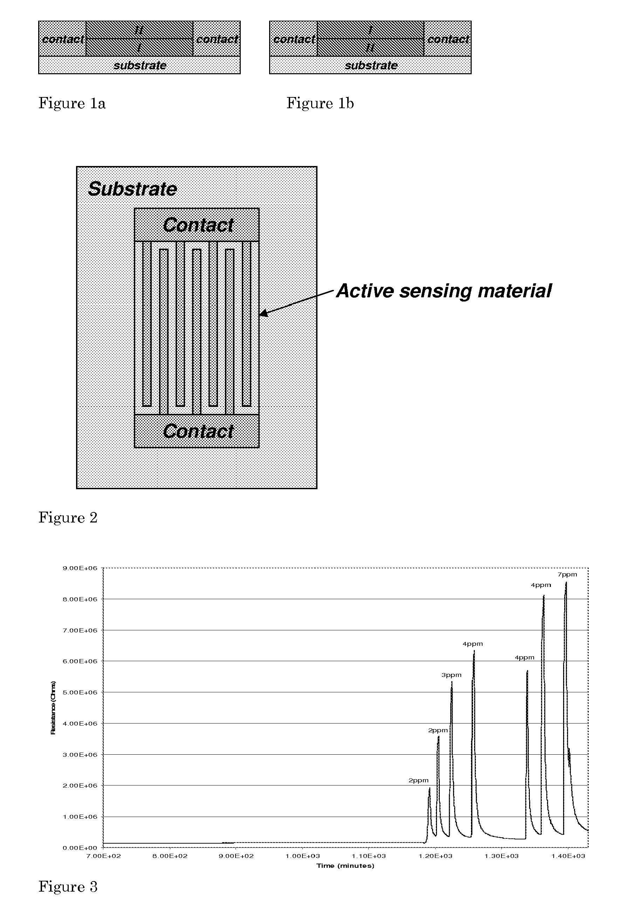

Preferably, the first (semiconducting) layer is positioned in between the substrate and the second (acidic) layer (see Figure 1a). Thus, the second layer shields the interface from the atmosphere and thus from the analyte. This configuration is in particular desirable if the second layer is also membrane. As a membrane, the second layer may either facilitate or make it more difficult for compounds, in particular the analyte and/or protons, to pass through the second layer. This may increase or decrease the sensitivity of the bilayer sensor. For these purposes, the bilayer may also be provided with a barrier layer, as explained below.

However, the second layer may also be positioned in between the first layer and the substrate (see Figure 1b).

By varying the properties of the second layer, the sensitivity of the sensor may be influenced, in particular as explained above in case the first layer is positioned in between the second layer and the substrate. For example, the sensitivity may be increased or decreased by varying the porosity of the second layer or by varying the thickness of the layer. By varying the porosity of the second layer, the analyte and/or protons may penetrate the second layer more difficult, thereby requiring a larger concentration of analyte to effectively dedope the interface of the bilayer. Thus, the sensitivity of the sensor may be increased. Furthermore, the second layer may comprise certain compounds that speed up or slow down proton transport and/or analyte penetration.

The first layer typically has a thickness of 10 nm to 1 µm, preferably a thickness of 50 nm to 20nm.

The second layer typically has a thickness of 50 nm to 10 µm, preferably a thickness of 100 nm to 1µm. As explained above, the thickness of the second layer may influence the sensitivity of the sensor.

The substrate of the sensor may be constructed from ceramics, such as SiO₂ and glass) or from polymeric supports, such as poly(ethylene naphthalate), poly(ethylene terephthalate), polyimide. Suitable substrates for sensors are generally known in the art. The skilled person will be able to choose a suitable substrate.

The substrate may further comprise electrical contacts.

The bilayer may further comprise two electrodes, which are separated from each other by the bilayer. These electrodes may be interdigitated electrodes (see Figure 2; the bilayer is referred to as "Active sensing material"). The electrodes may be made from any material known in the art to be suitable as such. For example, the electrodes can be fabricated from metal (Au, Pd, Pt, Ni, Cu, Al, etc.), conducting polymer, e.g. Poly(3,4-ethylenedioxythiophene) poly(styrenesulfonate) (PEDOT), or metal oxide, e.g. Tin doped Indium Oxide (ITO) and Aluminium doped Zinc Oxide (AZO).

The bilayer of the invention may comprise two or more layers. The first and second layer are preferably adjacent to each other. In case the bilayer comprises a barrier layer, such a layer may be positioned in between the first and the second layer.

The sensor of the invention may further comprise a barrier layer. A barrier layer is a layer that forms a barrier for certain compounds to pass through. The compounds for which the layer forms a barrier may for be the analyte, protons and/or compounds that may react with acidic or basic compound. The barrier layer may prevent or lower the chance that such compounds reach the second layer or the interface. Thus, the he properties of the barrier layer can provide for an increased control of the electrical and/or chemical properties of the bilayer. In particular, the barrier layer may provide a means for increasing or decreasing sensitivity and/or selectivity of the bilayer sensor.

The barrier layer is typically a polymer layer. Furthermore, the barrier layer is preferably a membrane. The barrier layer may for example be an ion or pH selective membrane or an acidic buffer layer.

The barrier layer may be positioned such that it forms a barrier between the bilayer and the atmosphere. This means that any analyte first needs to pass the barrier layer before it can reach the first and second layers. In such a case, the barrier layer does not need to be part of the bilayer.

The barrier layer may comprise ionophores, preferably amine or ammonium ionophores. Amine and ammonium ionophore are known in the art. Ionophores may influence the transporting properties of certain compounds through the barrier layer.

The barrier layer layer may also be a reactive layer, which is a layer that comprises compounds that react with the analyte. Due to such a reaction with the analyte, the barrier layer may reduce or increase the chance of the analyte reacting with the basic or acidic compound in the second layer, thus influencing the sensitivity of the bilayer. An example of a reactive layer is a selective buffer layer.

The barrier layer may also be a layer that influences the transport of compounds other than the analyte through the layer. For example, the barrier layer may be a membrane that is permeable for the analyte. Such a membrane may block other compounds that can influence the dedoping process. Thus, the selectivity of the sensor may be increased. Further properties of the barrier layer that can be used to control the sensitivity and/or selectivity of the buffer layer are the thickness of the barrier layer and the porosity of the barrier layer. Generally, it will be more difficult for compounds to pass through a thick unporous layer than through a thin porous layer.

The barrier layer typically has a thickness of 500 nm to 1 mm, preferably a thickness of 1 µm to 100 µm.

The bilayer sensor is preferably a disposable sensor intended for single use only. However, alternatively, the bilayer sensor may be a sensor that can be reused. In such a case, it needs to be possible to bring a bilayer that has been dedoped by the presence of amines or ammonia back to a state wherein the interface between the first and second layer is again doped with protons originating from the acid compounds in the second layer. Resetting of a base sensor can for example be conducted by bringing the sensor in contact with acid, which reverses the dedoping reaction. For example, the base sensor can be placed in an acidic environment for a certain amount of time, for example several hours. The acidic environment may be a container comprising an acidic aqueous solutions or an acidic gas such as hydrogen chloride (HCl). Suitable acids that can be used for in aqueous solutions are for example organic acids, *e.g.* acidic acid. Similarly, resetting of an acid sensor can be done by bringing the sensor in contact with a base.

In case of an acid sensor, the basic compound is preferably a polymeric compound, such as polyethyleneimide. However, the basic compound may also be a small organic compound. Such a small organic compound may be provided in a polymer matrix. Furthermore, the basic compound preferably comprises one or more amine groups. An example of a suitable semiconductor present in the first layer is a perylene-diimide derivative (Polyera N1400 active ink).

The sensor of the invention is particularly suitable for detecting gaseous analytes. However, it may also be used for detecting liquid analytes. In case the sensor of the invention is a base sensor, the sensor is in particular suitable for detecting amines and/or ammonia. In case the sensor of the invention is an acid sensor, the sensor may be used to detect acids, such as for example lactic acid.

The invention is further directed to a method for preparing the sensor of the invention. This method comprises the steps of
- applying a first layer to a substrate, and
- applying a second layer on top of the previous layer,
wherein either the first or second layer is a layer comprising a compound selected from the group consisting of acidic compounds and basic compounds, and
wherein the other layer is a layer comprising an organic semiconductor.

The semiconductor is preferably an intrinsic semiconductor. An intrinsic semiconductor is a semiconductor wherein no or at least no significant amount of a doping agent is present. Doping is the process of introducing impurities into a semiconductor, thereby changing the electrical properties of the semiconductor. A doping agent is an atom or molecule that is added in a semiconductor to increase the number of free charge carriers.

When the first and second layer are applied to each other, doping of the semiconductor will occur at the interface of the first and second layer.

The layers can be applied via various methods known in the art, such as printing (*e.g.* inkjet printing, offset printing, screen printing or gravure printing), spin coating, dip coating *in situ* polymerization and evaporation. Preferably, ink-jet or gravure printing is used to apply the layers, because of the large printing area and low costs.

Preferably, the first layer comprises an acidic compound, while the second layer comprises a semiconductor. However, the first layer may also comprise the semiconductor, while the second layer comprises the acidic compound.

The method may further comprise the additional step of applying a barrier layer. The barrier layer may be applied to the first layer or to the second layer. Consequently, the first or second layer will be applied on top of the barrier layer. Preferably, the barrier layer is applied such that the obtained sensor comprises the barrier layer positioned in between the first and second layer.

The specific details regarding the first layer, second layer, optional barrier layer and substrate described above for the sensor of the invention also apply to the method of the invention.

The invention is further directed to a food packaging comprising the bilayer sensor of the invention. Such a food packaging may for example comprise fish. The bilayer sensor may thus be used to monitor the freshness of fish. In such a case, the base sensor of the invention is used.

The food packaging may also comprise milk. In such a case, the acid sensor of the invention is preferably used.

The bilayer sensor of the invention can be suitably used in radio-frequency identification.

The bilayer of the invention can be used in various applications. The bilayer sensor may thus be used to monitor the freshness of food, in particular fish.

The sensor may further be used to monitor and/or analyze bacterial growth in a wound, for example in wound gas analysis. In wound gas analysis, the gas originating from a wound may be monitored to detect an infection in an early stage. The nitrous basic gases excreted by bacterial growth from a wound can be monitored using the bilayer sensor of the invention.

The invention will be illustrated by the following examples.

### Example 1: Effect of Ammonia on Resistance of the Bilayer Sensor

A bilayer was prepared comprising a first layer of poly(triarylamine) polymer and a second layer of sulfonated tetrafluorethylene polymer. The sulfonated tetrafluoroethylene polymers sold under the name Nafion.

The resistance of the bilayer was measured over a time period of about 16 hours under a relative humidity (RH) of 90%. During this time period the bilayer was exposed to different concentrations of ammonia.

First, the sensor was exposed to an environment of 2 ppm ammonia.

The sensor was subsequently reset to approximately its original resistance by extensively flushing the sensor and gas handling system recovery.

These two steps were repeated using an environment of 2 ppm, 3 ppm (three times) and 7 ppm ammonia respectively.

Figure 3 shows the results of the resistance measurements. Only minimal changes in resistance were observed when the sensor was not exposed to ammonia. When a concentration in the order of 2 ppm ammonia was applied a strong increase in resistance was observed (20 µL of 25% NH₃ diluted in 200 ml H₂O diluted a factor 10 with a 90% RH air flow yielded a concentration of approx 2 ppm). Re-exposing the sensor to the ammonia gas yields again a similar increase in resistivity. Some upward scatter in the measured data upon fast repeat of a measurement can be attributed to incomplete removal of the analyte from the gas flow system.

### Example 2: Sensitivitiy towards expired fish

A bilayer sensor comprising a first layer of poly(triarylamine) polymer and a second layer of fluorinated polystyrenesulphonate polymer was subsequently mounted into three different bottles each containing a different analyte.

First, the sensor was exposed to a mixture of diluted acidic acid in water (pH 3) for over 14 hours. Subsequently the sensor was exposed for 8 hours to a solution of demineralized water. Then, the same sensor was exposed to fouling fish.

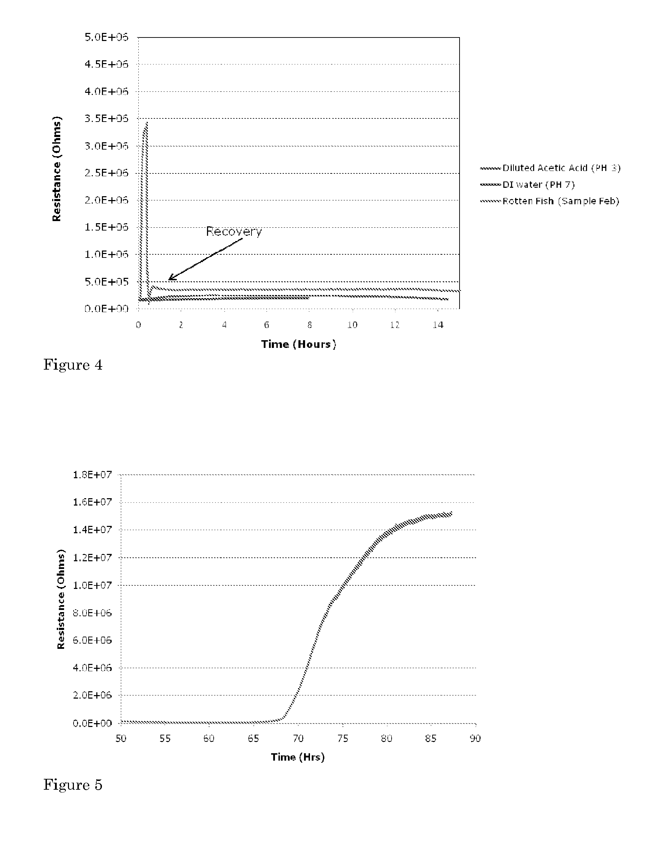

The results are depicted in Figure 4.

No substantial changes in resistance were observed for the acid and water analytes. When the same sensor was exposed for only minutes to fouling fish (1 month old fouling Salomon from sushi set AH), a strong response similar to that seen for ammonia was observed.

This example demonstrates the sensitivity of the bilayer sensor of the invention towards fouling fish. It is theorized that the sensitivity is caused by trimethylamine, which amine is excreted by fouling fish

### Example 3: Monitoring the Freshness of Fish

A bilayer sensor comprising a first layer of a triarylamine polymer and a second layer of a fluorinated polystyrenesulphonate polymer was exposed to a fresh piece of salmon (fresh sushi set AH), which was left at 21 °C for about three days. The resistance of the bilayer was measured over time. The results are shown in Figure 5.

For over 60 hours no response was observed. After 65 hours a slow and gradual increase in resistance was observed. It took over 10 hours before the sensor was fully saturated. After opening the bottle holding the fish, the presence of a fishy smell was observed by smelling, indicating the presence of trimethylamines.

Due to the suitable saturation times and strong but gradual response to trimethylamines, it can be concluded that the bilayer sensor can be suitably used for head space analysis of fresh fish.

### Example 4: Influence of Humidity on Reistance

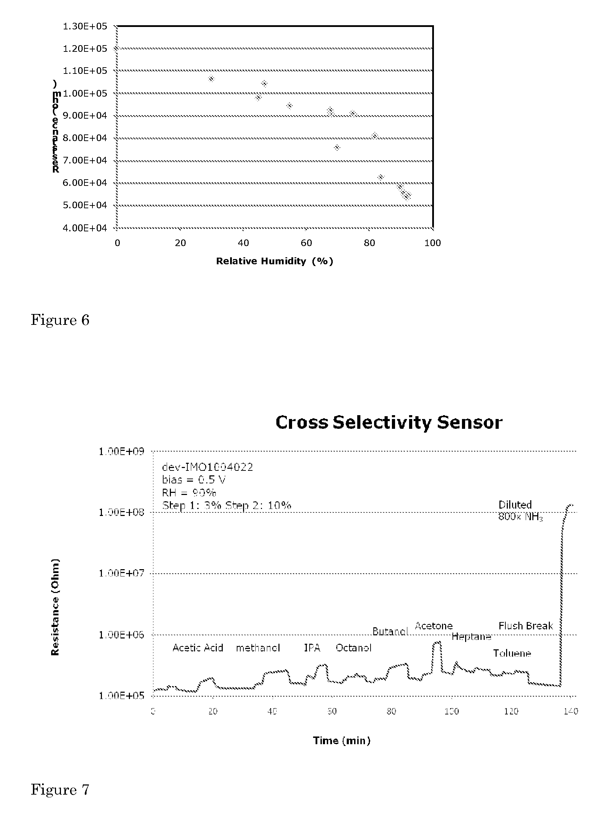

The resistance of the bilayer sensor was measured under varying relative humidity concentrations (from 0% to 90%). The results are shown in Figure 6.

The resistance varied from 120 kΩ at 0% humidity to 53 kΩ at 90% humidity. Thus, the resistance maximally changes by about a factor 2.3 Such changes are small compared to changes observed when contacted with amine or ammonia. For example, in example 2, a change from about 200 kΩ to about 3500 kΩ was observed, which corresponds to a factor 17.5.

From this example it can be concluded that the bilayer sensor shows a low sensitivity towards water. It exhibits stable operation even under extreme humidity conditions. Under a fixed (high) humidity, the baseline of the sensor remains stable in view of the eventual changes due to amine/ammonia contact.

### Example 5: Cross-Sensitivity

The cross-sensitivity of the bilayer was determined by comparing the resistance of the bilayer when brought into contact with a number of volatile organic compounds with the resistance of the bilayer when brought in contact with ammonia. The volatile organic compounds used were acetic acid, methanol, isopropanol (IPA), octanol, butanol, acetone, heptane and toluene. Measurements were conducted at a relative humidity of 90%. The compounds were tested using a gas having a concentration of about 3-10% of the saturated gas of the compound. The results are shown in Figure 7.

The resistance did not change much when the bilayer was contacted with the various volatile compounds (less than a factor 10). However, when a diluted solution of NH₃ was used the resistance increases by over a factor 100.

Thus, it can be concluded that the bilayer sensor of the invention has a relatively low cross sensitivity to various volatile organic compounds compared to amines and ammonia.

### Example 6: Sensitivity towards trimethylamine

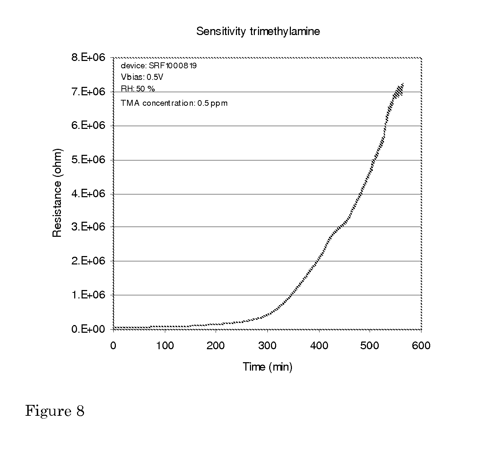

A bilayer sensor comprising of a first layer of a triarylamine polymer and a second layer a sulfonated tetrafluorethylene polymer is exposed to a constant flow of 0.5 ppm trimethylamine at a relative humidity of 50%. The results are shown in Figure 8. The sensors showed a gradual increase in resistance upon exposure to trimethylamine. The results confirm the sensors sensitivity towards small concenctrations of trimethylamine.

## Claims

1. Bilayer sensor comprising a substrate carrying a bilayer, which bilayer comprises a first layer comprising an organic semiconductor and a second layer comprising a compound selected from the group consisting of acidic compounds and basic compounds.

2. Bilayer sensor according to claim 1, wherein the second layer comprises an acidic compound.

3. Bilayer sensor according to claim 1 or 2, wherein the acidic compound is an acidic polymer.

4. Bilayer sensor according to any of the previous claims, wherein the acidic polymer is selected from the group consisting of acidic fluoropolymer-copolymer, polystyrenesulfonate and sulfonated tetrafluorethylene polymers.

5. Bilayer sensor according to any of the previous claims, wherein the semiconductor is a small organic compound selected from the group consisting of thiophenes, arylamines, acenes and pyrols

6. Bilayer sensor according to any of the previous claims, wherein the organic semiconductor is a material selected from the group consisting of polythiophenes, polyacenes, polyacrylamides, poly(triarylamine), polyfluorenes, and copolymers thereof.

7. Bilayer sensor according to any of the previous claims, wherein the second layer is positioned in between the substrate and the first layer.

8. Bilayer sensor according to any of the previous claims, wherein the sensor further comprises two electrodes separated from each other by the bilayer.

9. Bilayer sensor according to any of the previous claims, wherein the sensor comprises a barrier layer selected from the group consisting of ion and pH selective membranes, and wherein the barrier layer is preferably an amine and/or ammonium selective ionophore.

10. Food packaging comprising a sensor according to any of claims 1-9.

11. Method for preparing a bilayer sensor according to any of claims 1-9, comprising the steps of
- applying a first layer to a substrate, and
- applying a second layer on top of the first previous layer,
wherein either the first or second layer is a layer comprising a compound selected from the group consisting of acidic compounds and basic compounds, and wherein the other layer is a layer comprising an organic semiconductor.

12. Method according to claim 13, wherein the organic semiconductor is an intrinsic semiconductor.

13. Use of a sensor according to any of claims 1-12 in radio-frequency identification.

14. Use according to claim 16 for monitoring the freshness of fish.

15. Use of a sensor according to any of claims 1-12 for monitoring baterial growth in a wound.
